(19) Europäisches Patentamt
European Patent Office
Office européen des brevets

(11) **EP 4 177 905 A1**

(12) **EUROPEAN PATENT APPLICATION**

(43) Date of publication:
**10.05.2023 Bulletin 2023/19**

(21) Application number: **22162940.5**

(22) Date of filing: **18.03.2022**

(51) International Patent Classification (IPC):
*G16H 40/40* (2018.01)

(52) Cooperative Patent Classification (CPC):
**G16H 40/40**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **09.11.2021 US 202163277377 P**

(71) Applicant: **Koninklijke Philips N.V.
5656 AG Eindhoven (NL)**

(72) Inventors:
• **STOLIKJ, Milosh**
  **Eindhoven (NL)**
• **WANG, Lu**
  **Eindhoven (NL)**
• **GAO, Qi**
  **Eindhoven (NL)**

(74) Representative: **Philips Intellectual Property &
Standards
High Tech Campus 52
5656 AG Eindhoven (NL)**

(54) **SYSTEMS AND METHODS FOR EXTRACTING DIAGNOSTIC AND RESOLUTION PROCEDURES FROM HETEROGENOUS INFORMATION SOURCES**

(57) A non-transitory computer readable medium (26) stores instructions executable by at least one electronic processor (20) to perform a medical device servicing method (100) for a medical device (12), including: deriving a representation (36) of a base diagnostic procedure for diagnosing a malfunction of the medical device from one or more service manuals (32); analyzing historical service records (34) to extract diagnostic procedures applied in historical service cases for diagnosing the malfunction of the medical device; adding the extracted diagnostic procedures to the representation of the base diagnostic procedure to generate an updated representation (40) of the base diagnostic procedure; calculating likelihood of success scores (42) for each path of the updated representation of the base diagnostic procedure based on statistics of outcomes of the historical service cases; and outputting the updated representation of the base diagnostic procedure on an electronic processing device (18) operable by a service engineer (SE).

Fig. 2

100

Derive a representation of a base diagnostic procedure — 102

Extract diagnostic procedures from historical service records — 104

Update representation with extracted diagnostic procedures — 106

Calculate scores for paths of the updated representation — 108

Display updated representation — 110

**Description**

FIELD OF THE INVENTION

The following relates generally to the medical imaging arts, medical imaging device maintenance arts, medical imaging device diagnostic procedure arts, and related arts.

BACKGROUND OF THE INVENTION

A medical imaging system or other medical device may occasionally malfunction, and thus fail to work properly. The malfunction is recognized by an operator of the medical device based on observed symptoms, such as failure of the medical device to operate, unsatisfactory output of the medical device (e.g., a patient monitor unable to read a vital sign sensor, a medical imaging device producing low-quality images, or so forth), and/or by an error code produced by the medical device, or based on other symptoms. Depending on the malfunction, the problem can sometimes be resolved remotely, by a remote service engineer (RSE). During diagnosis, the RSE can interact with several tools that can aid during system troubleshooting, and for identifying the best course of action to resolve the problem. For example, the RSE may decide to perform several actions, such as searching through historical service records to find similar service cases, and identify how they were resolved, searching through technical documentation for possible root causes and potential solutions, inspecting the log files of the medical imaging system, to identify relevant diagnostic information such as error messages, contacting the customer to get a description of the problem, and so forth.

After the troubleshooting, the RSE should be able to determine the root cause of the problem and identify the right service solution. The service solution can be executed remotely (e.g., a software update, remote calibration, etc.) or on-site when some spare parts need to be replaced.

However, RSEs are often under time pressure to perform the troubleshooting and decide on the most appropriate service action. To make an informed decision, RSEs need to collect and examine relevant information from various sources. Among the plethora of sources, RSEs may access technical documentation, such as service manuals, installation manuals etc., which list potential symptoms, sequences of steps to perform diagnosis, and instructions on which service actions to take. However, as this is static documentation, it may not be complete, or it may not be up to date. Similarly, RSEs may access historical service records, to inspect how the same (or similar) problem was successfully resolved in the past by other RSEs. However, due to the complexity of problem and medical system as well as the amount information reported in both historical service records and technical documentation, it is challenging for the RSEs to quickly identify the most relevant information for the current problem and system configuration.

The following discloses certain improvements to overcome these problems and others.

SUMMARY OF THE INVENTION

In some embodiments disclosed herein, a non-transitory computer readable medium stores instructions executable by at least one electronic processor to perform a medical device servicing method for a medical device, including: deriving a representation of a base diagnostic procedure for diagnosing a malfunction of the medical device from one or more service manuals; analyzing historical service records to extract diagnostic procedures applied in historical service cases for diagnosing the malfunction of the medical device; adding the extracted diagnostic procedures to the representation of the base diagnostic procedure to generate an updated representation of the base diagnostic procedure; calculating likelihood of success scores for each path of the updated representation of the base diagnostic procedure based on statistics of outcomes of the historical service cases; and outputting the updated representation of the base diagnostic procedure on an electronic processing device operable by a service engineer (SE).

In some embodiments disclosed herein, an apparatus includes a display device, and at least one electronic processor programmed to: augment a representation of a base diagnostic procedure for diagnosing a malfunction of the medical device with diagnostic procedures applied in historical service cases for diagnosing the malfunction of the medical device to generate an updated representation of the base diagnostic procedure; calculate likelihood of success scores for each path of the updated representation of the base diagnostic procedure based on statistics of outcomes of the historical service cases; and output the updated representation of the base diagnostic procedure on the display device including at least a portion of the paths of the updated representation annotated by the calculated likelihood of success scores.

In some embodiments disclosed herein, a medical device servicing method includes: analyzing historical service records to extract diagnostic procedures applied in historical service cases for diagnosing a malfunction of the medical device; combining the extracted diagnostic procedures to generate a fault tree for diagnosing the malfunction of the medical device; labelling each path of the fault tree with a label comprising a number of historical service cases resolved by that path or with a likelihood of success score calculated based on the number of historical service cases resolved by that path, wherein each path comprises one or more routes or procedures performed by a service engineer for diagnosing a malfunction of the medical device; and outputting a representation of the fault tree including the labels. One advantage resides in supporting the servicing of a medical device by combining information from technical documentation and historical service records into a single diagnostic procedure view for display.

Another advantage resides in supporting the servicing of a medical device by building a diagnostic and resolution procedure from information collected from service manuals augmented with findings from historical service records.

Another advantage resides in enabling a service engineer to quickly judge the different diagnostic paths that could be taken, the potential service actions, and the likelihood of each path occurring and successfully resolving the problem, based on historical data.

Another advantage resides in providing a view of a combination of information from technical documentation and historical service records created interactively by service engineers, allowing the service engineers to request details of certain actions, and record the actions they performed.

Another advantage resides in automatically generating a service record report with a summary and logged interactions of a service engineer during a servicing process.

A given embodiment may provide none, one, two, more, or all of the foregoing advantages, and/or may provide other advantages as will become apparent to one of ordinary skill in the art upon reading and understanding the present disclosure.

BRIEF DESCRIPTION OF THE DRAWINGS

The disclosure may take form in various components and arrangements of components, and in various steps and arrangements of steps. The drawings are only for purposes of illustrating the preferred embodiments and are not to be construed as limiting the disclosure.

Fig. 1 diagrammatically illustrates a medical device servicing apparatus in accordance with the present disclosure.
Fig 2 diagrammatically illustrates medical device servicing method using the apparatus of Fig 1.
Figs 3-7 show examples outputs of the apparatus of Fig 1.

DETAILED DESCRIPTION OF EMBNODIMENTS

The following relates to providing an improved diagnostic procedure recommender system based on published service manuals and historical service records.

Initially, a base diagnostic procedure for diagnosing the malfunction of the medical device is provided electronically, or is extracted by natural language processing (NLP) or computer vision (CV) from a published service manual. The service manual may, for example, be in binary (e.g., PDF) format, or in a structured machine-readable format. For the binary format manuals, optical character recognition (OCR) and NLP are applied to extract diagnostic procedure information from text, while these tools along with CV are applied to extract diagnostic procedure information from flowcharts or other diagrams. In one approach, parts of the manual are classified by subsystem, error code, and/or other classification schema to facilitate retrieving relevant parts for specific diagnostic tasks. The base diagnostic procedure is suitably represented as a diagnostic tree, although other representations such as a diagnostic map are also contemplated. Alternatively, in some other embodiments the base diagnostic may be provided directly, e.g. as a data structure representing the base diagnostic procedure as fault tree, or may be omitted entirely.

Next, historical service records are analyzed by NLP and/or based on structure of the service reports to extract diagnostic procedures that were actually applied by service engineers (SEs) in diagnosing the malfunction of the medical device under consideration. The extracted diagnostic procedures are expected to include numerous instances of the base diagnostic procedure outlined in the service manual, but may also include variant diagnostic procedures or even wholly new diagnostic procedures developed by service engineers to resolve specific historical cases. Statistics are also developed on the number of historical cases resolved by each of those diagnostic procedures.

The base diagnostic procedure is then augmented by additional paths corresponding to other diagnostic procedures (besides the base procedure) that were extracted from the historical service records, and each path (including both those of the base procedure and any variant or wholly different procedures extracted from the historical records) is labeled with number of historical cases resolved by that path. This produces an augmented diagnostic procedure with typically more paths than the base diagnostic procedure. As used herein, the term "path" (and variants thereof) refers to one or more routes or procedures performed by a service engineer for diagnosing a malfunction of a medical device. In other embodiments, the base diagnostic procedure may be omitted entirely, and the extracted diagnostic procedures combined to generate a fault tree for diagnosing the malfunction of the medical device, which serves in place of the augmented diagnostic procedure in the subsequent steps.

To apply the augmented diagnostic procedure in a current case, likelihood of success scores are assigned to the various paths of the procedure. An example scoring formula for a path comprises a weighted sum of factors including: (i) the number of cases resolved by that path; (ii) resolution quality, e.g., time to solution or customer satisfaction feedback; and (iii) similarity of the path to the particulars of the current case. In another alternatively, each path may be labeled with the number of cases resolved by that path.

Finally, a user interface (UI) is provided. In one embodiment, the UI could display the entire augmented diagnostic procedure with all available diagnostic paths labeled with the scores. However, this may be too complex for easy consumption by a time-pressed service engineer. In another (not necessarily mutually exclusive) embodiment, a ranked summary of the highest scoring paths is provided, perhaps with links to bring up the portions of the augmented diagnostic procedure including those highest-ranked paths.

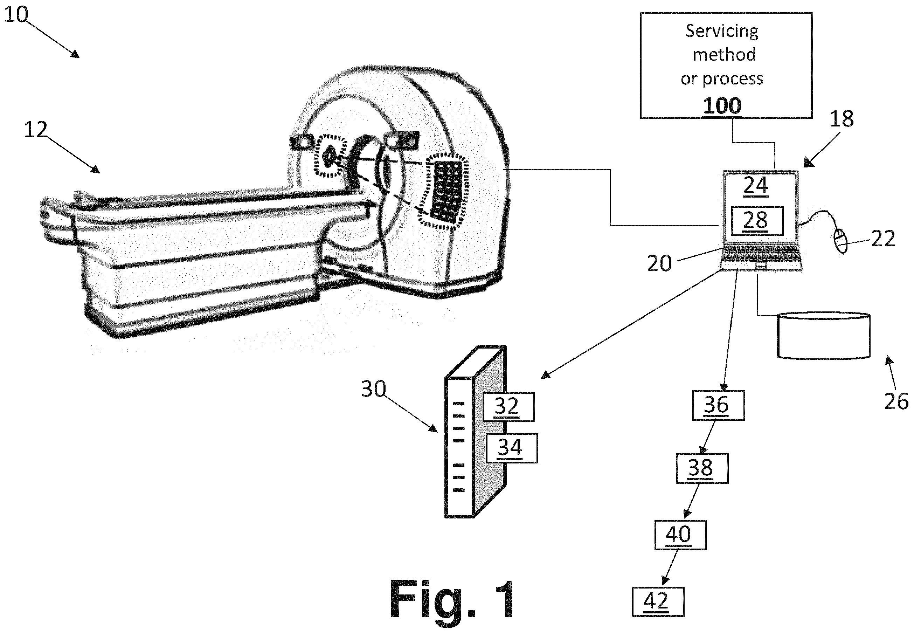

With reference to Fig 1, an illustrative apparatus **10** for servicing a medical device **12** is shown. The medical device **12,** for example, can comprise a medical imaging device **12** (also referred to as a medical device, an imaging device, imaging scanner, and variants thereof) can be a magnetic resonance imaging (MRI) scanner, a computed tomography (CT) scanner, a positron emission tomography (PET) scanner, a gamma camera for performing single photon emission computed tomography (SPECT), an interventional radiology (IR) device, an X-ray device, an image guided therapy (iGT) device, or so forth. Although shown in Fig 1 as an imaging device, the medical device **12** can also be any other suitable medical device, such as a patient monitor, a radiation therapy device, a mechanical ventilator, and so forth.

An electronic processing device **18,** such as a workstation computer, or more generally a computer, a smart device (e.g., a cellular telephone ("cell phone"), a smart tablet, and so forth), is operable by a service engineer (SE). The electronic processing device **18** may also include a server computer or a plurality of server computers, e.g., interconnected to form a server cluster, cloud computing resource, or so forth, to perform more complex computational tasks. The electronic processing device **18** includes typical components, such as an electronic processor **20** (e.g., a microprocessor), at least one user input device (e.g., a mouse, a keyboard, a trackball, and/or the like) **22,** and a display device **24** (e.g., an LCD display, plasma display, cathode ray tube display, and/or so forth). In some embodiments, the display device **24** can be a separate component from the electronic processing device **18,** or may include two or more display devices.

The electronic processor **20** is operatively connected with one or more non-transitory storage media **26.** The non-transitory storage media **26** may, by way of non-limiting illustrative example, include one or more of a magnetic disk, RAID, or other magnetic storage medium; a solid-state drive, flash drive, electronically erasable read-only memory (EEROM) or other electronic memory; an optical disk or other optical storage; various combinations thereof; or so forth; and may be for example a network storage, an internal hard drive of the workstation **18,** various combinations thereof, or so forth. It is to be understood that any reference to a non-transitory medium or media **26** herein is to be broadly construed as encompassing a single medium or multiple media of the same or different types. Likewise, the electronic processor **20** may be embodied as a single electronic processor or as two or more electronic processors. The non-transitory storage media **26** stores instructions executable by the at least one electronic processor **20.** The instructions include instructions to generate a visualization of a graphical user interface (GUI) **28** for display on the display device **24.**

The electronic processing device **18** is also in communication with a database **30** (shown in Fig 1 as a server computer) that stores service manuals **32** related to diagnostic procedures for a plurality of different medical devices (including the medical device **12** shown in Fig 1). The database **30** also stores historical service records **34** related to previously-performed diagnostic procedures for diagnosing various malfunctions for a plurality of different medical devices (possibly including the medical device **12** shown in Fig 1).

The apparatus **10** is configured as described above to perform a medical device servicing method or process **100.** The non-transitory storage medium **26** stores instructions which are readable and executable by the at least one electronic processor **20** to perform disclosed operations including performing the servicing method or process **100.** In some examples, the method **100** may be performed at least in part by cloud processing.

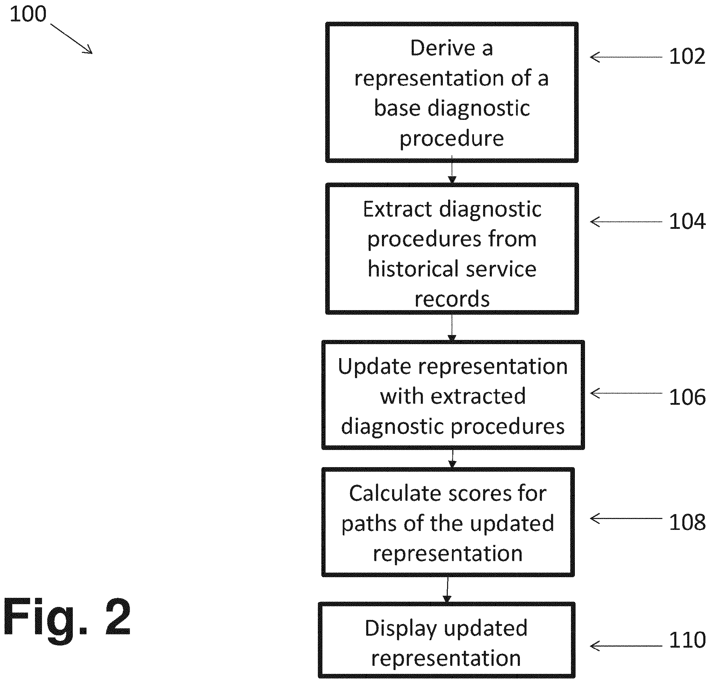

With reference to Fig 2, and with continuing reference to Fig 1, an illustrative embodiment of an instance of the servicing method **100** is diagrammatically shown as a flowchart. At an operation **102,** a representation **36** of a base diagnostic procedure for diagnosing a malfunction of the medical device **12** is derived from one or more service manuals **32** retrieved from the database **30.** The malfunction may be recognized in terms of specific symptoms, one or more error messages, observed deficiencies in the operation of the medical device **12,** failure of a component of the medical device **12** to operate at all, and/or so forth. The base diagnostic procedure includes information such as problem symptoms indicative of the malfunction, potential root cause of the malfunction, error code(s) shown in a user interface, error code(s) logged in system (i.e., debug) log files, diagnostic steps to take to troubleshoot the problem, including expected outcomes, service actions to take to resolve the problem, and so forth. The representation **36** of the base diagnostic procedure can include, for example, a diagnostic tree, a diagnostic map, or any other suitable representation.

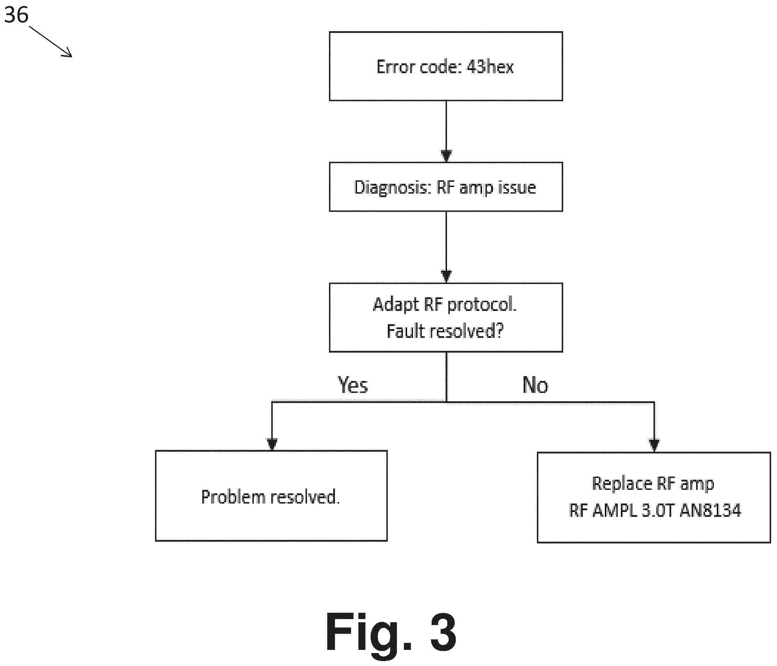

To derive the representation **36,** a suitable process can be performed on the service manual(s) **32,** such as natural language processing (NLP) process (i.e., when the information in the service manual(s) **32** is textual), a computer vision (CV) process (i.e., when the information in the service manual(s) **32** is graphical), manually, and so forth. For example, for an error code (e.g., "43hex" as one nonlimiting illustrative example) can be associated with two possible outcomes: either a scan protocol used is incorrect, or a component (i.e., a radiofrequency (RF) amplifier) is broken. Fig 3 shows an example of the representation **36** of a base diagnostic procedure for an error code 43hex.

In some other embodiments, the representation **36** of the base diagnostic procedure for diagnosing the malfunction of the medical device can be provided directly, rather than being derived from service manual(s). For example, the representation **36** of the base diagnostic procedure may be provided in a directly consumable electronic form such as a data structure representing the base diagnostic procedure as fault tree. In such embodiments, the operation **102** is suitably omitted, or alternatively may be replaced by a retrieval operation that retrieves the data structure representing the fault tree.

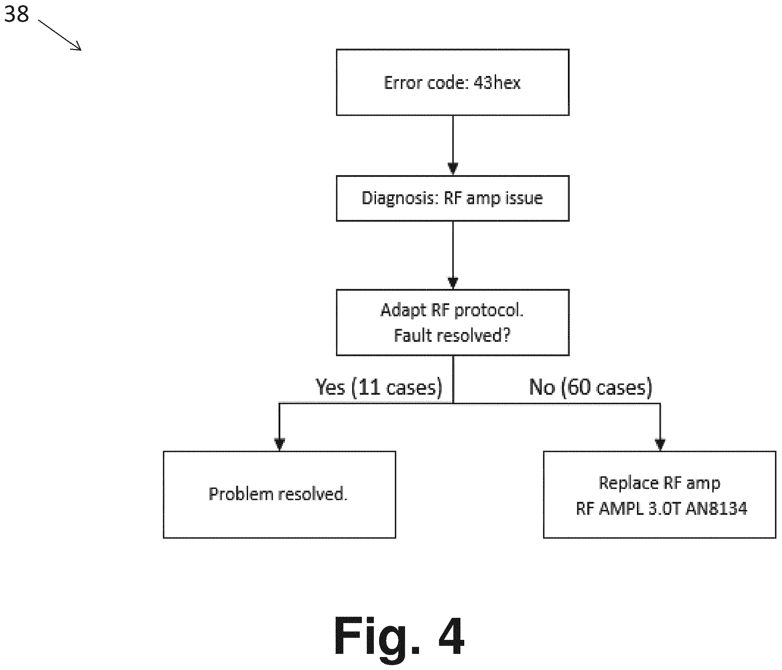

At an operation **104,** one or more historical service records **34** (retrieved from the database **30**) are analyzed to extract diagnostic procedures **38** applied in historical service cases for diagnosing the malfunction of the medical device **12.** To extract the diagnostic procedures **38,** a suitable process can be performed on the historical service record (s) **34,** such as natural language processing (NLP) process, a computer vision (CV) process, and so forth. In a typical scenario, the historical service records **34** are in the form of structured service reports for historical service cases handled by service engineers. In this scenario, the relevant content may be extracted from structured and/or freeform fields of the service reports, such as a "service actions" field, a "diagnostic tests" field, and/or so forth. Real-time statistics on a number of times each procedure was used, what was the success rate of each taken step, which procedures were explored at the same time, and so forth, can be determined from the number of historical service records **34** that follow each procedure and the results indicated in the historical service records **34.** Fig 4 shows an example of the diagnostic procedure **38** for the error code 43hex.

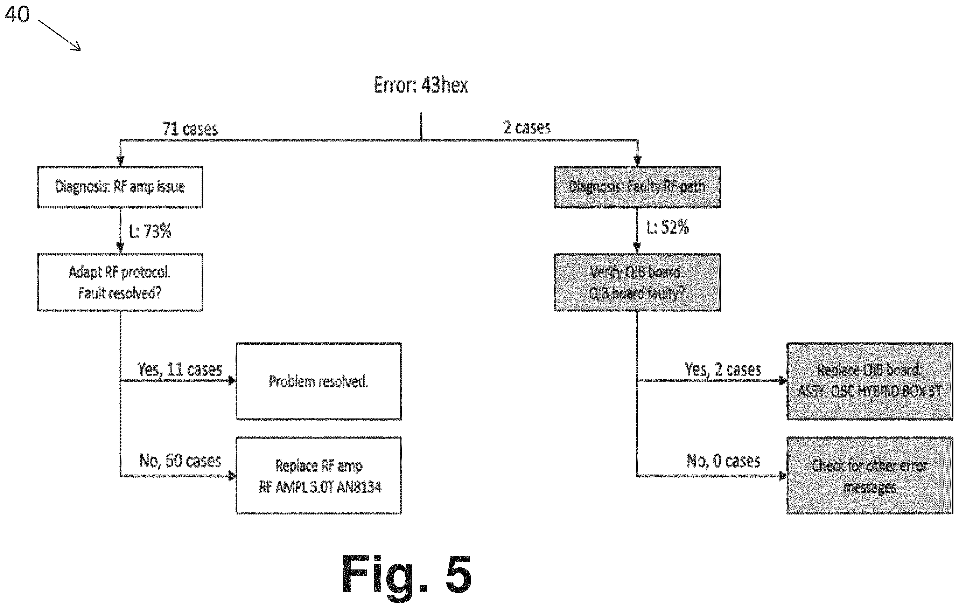

At an operation **106,** the extracted diagnostic procedures are added to the representation **36** of the base diagnostic procedure to generate an updated representation **40** of the base diagnostic procedure. For example, common nodes of the extracted diagnostic procedures and the base diagnostic procedure are mapped, and nodes of the extracted diagnostic procedures that are not contained in the base diagnostic procedure are added. Furthermore, each path of the updated representation **40** of the base diagnostic procedure are labelled with a number of historical service cases **34** resolved by that path (or, in another embodiment, with a likelihood of success computed based on that path). Fig 5 shows an example of the updated representation **40** of the base diagnostic procedure for the error code 43hex with the numbers of historical service cases **34** annotated to the paths. In some embodiments, the updated representation **40** can include adding additional paths described in number of historical service cases **34.**

In some other embodiments, the representation **36** of the base diagnostic procedure is omitted entirely, and the operation **106** combines the extracted diagnostic procedures to generate a fault tree for diagnosing the malfunction of the medical device. In these embodiments, the fault tree constructed by combining the extracted diagnostic procedures (without starting from a base diagnostic procedure) corresponds to the updated representation **40** of the base diagnostic procedure in the subsequent operations.

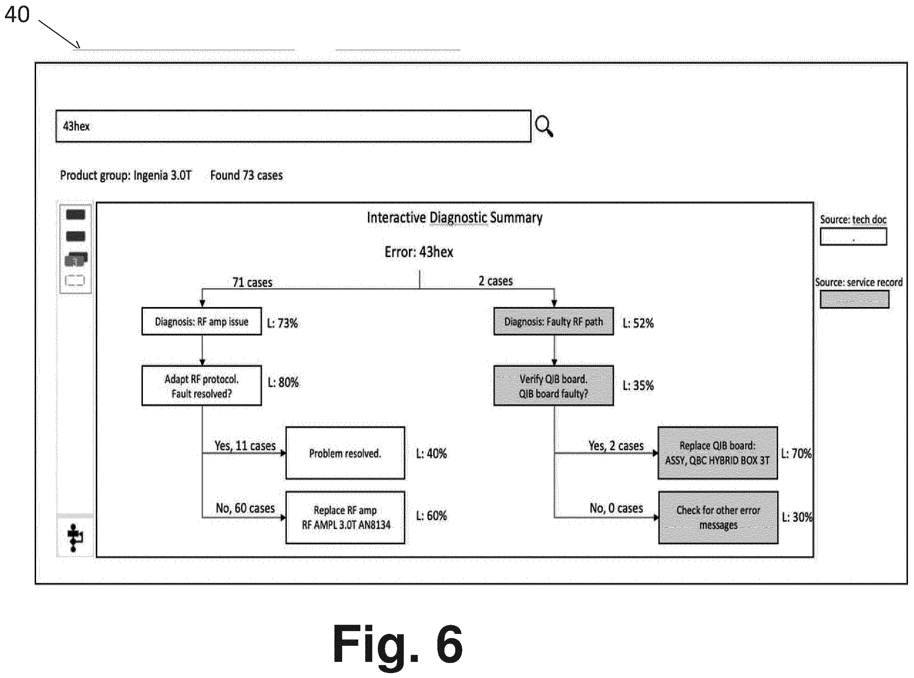

At an operation **108,** likelihood of success scores **42** are calculated for each path of the updated representation **36** of the base diagnostic procedure based on statistics of outcomes of the historical service cases **34.** (As previously noted, these can be used in place of the number of historical cases as the annotations for the updated representation **36**). The likelihood of success scores **42** can indicate how likely that a service action will lead to successful service resolution for the given malfunction reported by the customer. Fig 6 shows an example of the updated representation **40** of the base diagnostic procedure with the calculated likelihood of success scores **42** annotated to the paths for the updated representation **36** in which the error code 43hex is an observed symptom of the malfunction.

The likelihood of success scores **42** can be calculated based on one or more factors, including a number of historical service cases resolved by each path of the updated representation **40** of the base diagnostic procedure, outcome quality statistics of historical service cases **34** resolved by each path of the updated representation **40** of the base diagnostic procedure, a similarity metric of each path of the updated representation **40** of the base diagnostic procedure relative to the current service case, and so forth. In another embodiment, the likelihood of success scores **42** can be calculated based on a weighted sum of these three described factors.

In a particular example, given a problem p, a set of related cases C, a service action $s_i$ and a set of cases where that service action was performed, $C_{s_i}$, the score can be calculated according to Equation 1:

$$score(s_i, p) = w_1 \frac{|C_{s_i}|}{|C|} + w_2 \sum_{c_j \in C_{s_i}} q(c_j) + w_3\, sim(s_i, p) \qquad (1)$$

where $w_1$, $w_2$ and $w_3$ are weights for each factor described previously, q is a function that determines the quality-of-service delivery for a given case, and $sim(s_i, p)$ is a similarity indicator of the problem description to the service action.

At an operation **110,** the updated representation **40** of the base diagnostic procedure is output on, for example, on the display device **24** of the electronic processing device **18** via the GUI **28.** In some embodiments, the displayed updated representation **40** can include the updated representation **40** with the likelihood of success scores **42** annotating the respective paths. In other embodiments, the displayed updated representation **40** can include the updated representation **40** as a ranked summary of paths having high likelihood of success scores **42.**

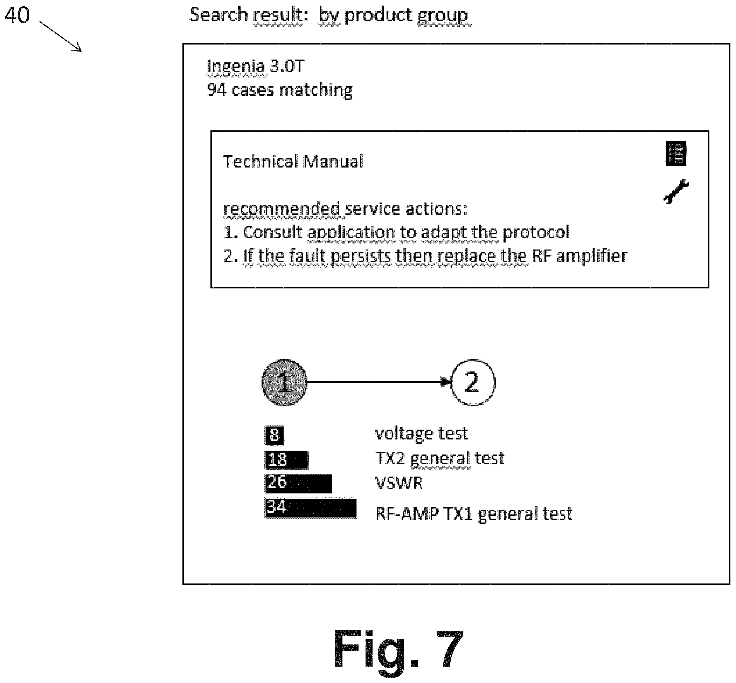

In some embodiments, as shown in Fig 7, the displayed updated representation **40** can comprise a diagnostic tree (or can comprise nay other suitable representation such as a map with a bar chart). The RSE can then search the displayed updated representation **40** for an error code (i.e., 43hex). Referring back to Fig 6, the updated representation **40** shows the result from both technical documentation (labelled in white rectangles) and service records (labelled in grey rectangles). The labels such as 'L: 73%' and 'L: 52%' indicate the corresponding likelihood of success. In this way, the RSE can see the source of information, as well as the predicted success rate. Optionally, if RSEs request the details of a certain action, then the RSEs can click it. From there, RSEs can navigate to the corresponding parts of the documentation, inspect the associate service cases, or trigger an analysis of the available log data from the medical device **12.**

Once the RSE finishes following certain actions from the interactive diagnostic procedure, and assuming they the selected actions are selected from the displayed updated representation **40,** a service record report can be automatically generated for the RSE for verification and stored as a historical service record **34** in the database **30.**

The disclosure has been described with reference to the preferred embodiments. Modifications and alterations may occur to others upon reading and understanding the preceding detailed description. It is intended that the exemplary embodiment be construed as including all such modifications and alterations insofar as they come within the scope of the appended claims or the equivalents thereof.

**Claims**

1. A non-transitory computer readable medium **(26)** storing instructions executable by at least one electronic processor **(20)** to perform a medical device servicing method **(100)** for a medical device **(12),** the method comprising:

   deriving a representation **(36)** of a base diagnostic procedure for diagnosing a malfunction of the medical device from one or more service manuals **(32);**
   analyzing historical service records **(34)** to extract diagnostic procedures applied in historical service cases for diagnosing the malfunction of the medical device;
   adding the extracted diagnostic procedures to the representation of the base diagnostic procedure to generate an updated representation **(40)** of the base diagnostic procedure;
   calculating likelihood of success scores **(42)** for each path of the updated representation of the base diagnostic procedure based on statistics of outcomes of the historical service cases; and
   outputting the updated representation of the base diagnostic procedure on an electronic processing device **(18)** operable by a service engineer (SE).

2. The non-transitory computer readable medium **(26)** of claim 1, wherein adding the extracted diagnostic procedures to the representation **(36)** of the base diagnostic procedure to generate an updated representation **(40)** of the base diagnostic procedure includes:
   labelling each path of the updated representation of the base diagnostic procedure with a number of historical service cases resolved by that path.

3. The non-transitory computer readable medium **(26)** of either one of claims 1 and 2, wherein adding the extracted diagnostic procedures to the representation **(36)** of the base diagnostic procedure to generate an updated representation **(40)** of the base diagnostic procedure includes:
   adding additional paths described in the historical service records **(34)** to_the updated representation.

4. The non-transitory computer readable medium **(26)** of any one of claims 1-3, wherein calculating likelihood of success scores **(42)** for each path of the updated representation **(40)** of the base diagnostic procedure includes:
   calculating the likelihood of success scores based on a number of historical service cases resolved by each path.

5. The non-transitory computer readable medium **(26)** of any one of claims 1-4, wherein calculating likelihood of success scores **(42)** for each path of the updated representation **(40)** of the base diagnostic procedure includes:
   calculating the likelihood of success scores based on outcome quality statistics of historical service cases resolved by each path.

6. The non-transitory computer readable medium **(26)** of any one of claims 1-5, wherein the likelihood of success scores are calculated with respect to a current service case and calculating likelihood of success scores **(42)** for each path of the updated representation **(40)** of the base diagnostic procedure includes:
calculating the likelihood of success scores based on a similarity metric of each path relative to the current service case.

7. The non-transitory computer readable medium **(26)** of any one of claims 1-6, wherein analyzing historical service records **(34)** to extract diagnostic procedures applied in historical service cases for diagnosing the malfunction of the medical device **(12)** includes:
performing a natural language processing (NLP) process on the historical service records.

8. The non-transitory computer readable medium **(26)** of any one of claims 1-7, wherein outputting the updated representation **(40)** of the base diagnostic procedure on an electronic processing device **(18)** operable by a SE includes:
displaying the updated representation with the likelihood of success scores **(42)** annotating the respective paths.

9. The non-transitory computer readable medium **(26)** of any one of claims 1-8, wherein outputting the updated representation **(40)** of the base diagnostic procedure on an electronic processing device **(18)** operable by a SE includes:
displaying the updated representation as a ranked summary of paths having high likelihood of success scores **(42)**.

10. The non-transitory computer readable medium **(26)** of any one of claims 1-9, wherein the representation **(36)** of a base diagnostic procedure comprises one of a diagnostic tree or a diagnostic map.

11. The non-transitory computer readable medium **(26)** of any one of claims 1-10, wherein retrieving a representation **(36)** of a base diagnostic procedure for diagnosing the malfunction of the medical device from one or more service manuals **(32)** includes:
performing a natural language processing (NLP) process on the one or more service manuals.

12. The non-transitory computer readable medium **(26)** of any one of claims 1-10, wherein retrieving a representation **(36)** of a base diagnostic procedure for diagnosing the malfunction of the medical device from one or more service manuals **(32)** includes:
performing a computer vision (CV) process on the one or more service manuals.

13. The non-transitory computer readable medium **(26)** of any one of claims 1-12, wherein each path of the updated representation **(40)** of the base diagnostic procedure comprises one or more routes or procedures performed by the SE for diagnosing a malfunction of the medical device **(12).**

14. An apparatus **(10),** comprising:

a display device **(24);** and
at least one electronic processor **(20)** programmed to:

augment a representation **(36)** of a base diagnostic procedure for diagnosing a malfunction of the medical device with diagnostic procedures applied in historical service cases for diagnosing the malfunction of the medical device to generate an updated representation **(40)** of the base diagnostic procedure;
calculate likelihood of success scores **(42)** for each path of the updated representation of the base diagnostic procedure based on statistics of outcomes of the historical service cases; and
output the updated representation of the base diagnostic procedure on the display device including at least a portion of the paths of the updated representation annotated by the calculated likelihood of success scores.

15. The apparatus **(10)** of claim 14, wherein the likelihood of success scores **(42)** are calculated as a weighted sum of a number of historical service cases resolved by each path, outcome quality statistics of historical service cases resolved by each path, and a similarity metric of each path relative to the current service case.

Servicing method or process **100**

Fig. 1

**Fig. 2**

100

Derive a representation of a base diagnostic procedure ← 102

Extract diagnostic procedures from historical service records ← 104

Update representation with extracted diagnostic procedures ← 106

Calculate scores for paths of the updated representation ← 108

Display updated representation ← 110

36

**Fig. 3**

EP 4 177 905 A1

Fig. 4

**Fig. 5**

40

Error: 43hex

71 cases

2 cases

Diagnosis: RF amp issue

Diagnosis: Faulty RF path

L: 73%

L: 52%

Adapt RF protocol.
Fault resolved?

Verify QIB board.
QIB board faulty?

Yes, 11 cases

Problem resolved.

Yes, 2 cases

Replace QIB board:
ASSY, QBC HYBRID BOX 3T

No, 60 cases

Replace RF amp
RF AMPL 3.0T AN8134

No, 0 cases

Check for other error
messages

EP 4 177 905 A1

12

Fig. 6

40

Search result: by product group

Ingenia 3.0T
94 cases matching

Technical Manual

recommended service actions:
1. Consult application to adapt the protocol
2. If the fault persists then replace the RF amplifier

| 8 | voltage test |
| 18 | TX2 general test |
| 26 | VSWR |
| 34 | RF-AMP TX1 general test |

# Fig. 7

EP 4 177 905 A1

Europäisches
Patentamt

European
Patent Office

Office européen
des brevets

# EUROPEAN SEARCH REPORT

**Application Number**

EP 22 16 2940

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/215125 A1 (YOKOI MOTOHISA [JP] ET AL) 20 November 2003 (2003-11-20) * paragraph [0034] – paragraph [0097] * ----- | 1-15 | INV. G16H40/40 |
| A | WO 2020/117733 A1 (BECKMAN COULTER INC [US]) 11 June 2020 (2020-06-11) * paragraph [0008] – paragraph [0044] * ----- | 1-15 | |
| A | US 2014/136259 A1 (KINSEY II EDWARD PHILLIP [US] ET AL) 15 May 2014 (2014-05-15) * paragraph [0209] – paragraph [0471] * ----- | 1-15 | |
| A | US 2018/247023 A1 (DIVINE LUCAS JASON [US] ET AL) 30 August 2018 (2018-08-30) * paragraph [0026] – paragraph [0184] * ----- | 1-15 | |

**TECHNICAL FIELDS SEARCHED (IPC)**

G16H

The present search report has been drawn up for all claims

| Place of search | Date of completion of the search | Examiner |
|---|---|---|
| Munich | 13 July 2022 | Rinelli, Pietro |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 16 2940

This annex lists the patent family members relating to the patent documents cited in the above-mentioned European search report.
The members are as contained in the European Patent Office EDP file on
The European Patent Office is in no way liable for these particulars which are merely given for the purpose of information.

13-07-2022

| Patent document cited in search report | | Publication date | Patent family member(s) | | Publication date |
|---|---|---|---|---|---|
| US 2003215125 | A1 | 20-11-2003 | US 2003215125 A1 | | 20-11-2003 |
| | | | US 2005234330 A1 | | 20-10-2005 |
| WO 2020117733 | A1 | 11-06-2020 | NONE | | |
| US 2014136259 | A1 | 15-05-2014 | US 2014136259 A1 | | 15-05-2014 |
| | | | US 2014136264 A1 | | 15-05-2014 |
| | | | US 2014136265 A1 | | 15-05-2014 |
| | | | US 2014136266 A1 | | 15-05-2014 |
| | | | US 2014136373 A1 | | 15-05-2014 |
| | | | US 2014136443 A1 | | 15-05-2014 |
| | | | US 2020184408 A1 | | 11-06-2020 |
| | | | US 2021224723 A1 | | 22-07-2021 |
| | | | WO 2014078672 A2 | | 22-05-2014 |
| US 2018247023 | A1 | 30-08-2018 | CN 110301012 A | | 01-10-2019 |
| | | | EP 3586253 A1 | | 01-01-2020 |
| | | | EP 3703070 A1 | | 02-09-2020 |
| | | | JP 6949128 B2 | | 13-10-2021 |
| | | | JP 2020510915 A | | 09-04-2020 |
| | | | JP 2021099866 A | | 01-07-2021 |
| | | | US 2018247023 A1 | | 30-08-2018 |
| | | | US 2018247024 A1 | | 30-08-2018 |
| | | | US 2021225497 A1 | | 22-07-2021 |
| | | | WO 2018156192 A1 | | 30-08-2018 |

EPO FORM P0459

For more details about this annex : see Official Journal of the European Patent Office, No. 12/82